# EUROPÄISCHE PATENTSCHRIFT

(11) **EP 3 902 576 B1**
(45) Veröffentlichungstag und Bekanntmachung des Hinweises auf die Patenterteilung: **04.12.2024**
(21) Anmeldenummer: 19842863.3
(22) Anmeldetag: 27.12.2019
(51) Int. Cl.: A61M 1/00, A61B 17/00, A61B 17/32, A61M 3/02, A61B 1/12

(54) **MEDIZINTECHNISCHES GERÄT FÜR DIE MINIMAL-INVASIVE THERAPIE MIT MINDESTENS ZWEI GETRENNTEN SAUGLINIEN**
MEDICAL INSTRUMENT FOR MINIMALLY INVASIVE THERAPY, COMPRISING AT LEAST TWO SEPARATE SUCTION LINES
APPAREIL MÉDICAL DESTINÉ AU TRAITEMENT MINIMALEMENT INVASIF COMPORTANT AU MOINS DEUX LIGNES D'ASPIRATION SÉPARÉES

(30) Priorität: 28.12.2018 DE 102018010008
(43) Veröffentlichungstag der Anmeldung: 03.11.2021
(73) Patentinhaber: W.O.M. World of Medicine GmbH, 10587 Berlin (DE)
(72) Erfinder: SCHNÜTTGEN, Stan, 10179 Berlin (DE)
(74) Vertreter: Gulde & Partner
(86) Internationale Anmeldenummer: PCT/DE2019/000336
(87) Internationale Veröffentlichungsnummer: WO 2020/135900

(56) Entgegenhaltungen:
- WO-A1-2017/095679
- WO-A1-93/17729
- DE-A1- 10 233 053
- DE-A1- 102018 211 347
- DE-B4- 10 233 053
- FR-A1- 2 727 847
- US-A1- 2008 243 054
- US-B1- 6 396 583

## Beschreibung

### Erfindungsgegenstand

Medizintechnisches Gerät zum Einsatz in der minimal-invasiven Chirurgie mit mindestens zwei verschiedenen, unabhängig voneinander ansteuerbaren Sauglinien. Bevorzugt weisen die Sauglinien eine unterschiedliche Saugcharakteristik auf.

### Stand der Technik

In der minimal-invasiven Chirurgie wird häufig eine Kavität (natürliche oder künstlich geschaffene Körperhöhle) aufgedehnt, indem ein Fluid eingebracht wird, welches die Kavität unter Druck setzt und die Aufdehnung so ermöglicht. Durch eingebrachte medizintechnische Instrumente (siehe unten) wird eine therapeutische Behandlung durchgeführt, zum Beispiel die Glättung eines Knorpels. Beispielhaft wird eine derartige Vorrichtung in der EP 1382291 A2 gezeigt. hierfür wird in Dabei wird bestimmungsgemäß durch das jeweilige medizintechnische Instrument dieses Fluid abgesaugt, um deren Funktion sicherzustellen bzw. die Nebenwirkungen auf die aufgedehnten Kavität zu minimieren. Beispiel für solche medizintechnischen Instrumente sind Instrumente mit bewegten Klingen, bei denen die Absaugung das abzutrennende Gewebe heranzieht und die Gewebereste aus der Kavität entfernt (Shaver, Morcellator) und dann außerhalb der Kavität wegbefördert. Ein weiteres Beispiel für solche medizintechnische Instrumente sind die mit hochfrequentem Strom betriebenen Abtragsvorrichtungen (HF-Instrumente), bei denen die entstehenden Nebenprodukte der Anwendung wie Wasserdampf oder Rauchgase absaugt werden. Zusätzlich kann die entstehende Wärme mittels einer Absaugung des Fluids durch den Instrumentenkörper aus der Kavität abgeführt werden. Um den Druck und darüber die Aufdehung der Kavität aufrecht erhalten zu können, wird durch eine angepasste Regelung die Zufuhr des Fluids zur Kavität entsprechend, das heißt um mindestens das zusätzlich abgesaugte Volumen, erhöht. Diese Nachführung des abgesaugten Fluides ist schwierig, da die über die medizintechnischen Instrumente abgesaugten Mengen bzw. die Zeitpunkte der Absaugung durch das Öffnen von Ventilen am Handstück durch den Operateur bzw. gekoppelt über Ventile an dem medizintechnischen Instrument erfolgt und dem medizintechnischen Gerät zur Bereitstellung eines Fluidmanagement nicht über Sensoren bekannt sind. Typischerweise haben medizintechnische Geräte zur Bereitstellung des Fluidmanagements nur auf der Zufuhrseite einen Drucksensor, der über die Fluidkommunikation entlang der in der Kavität endenden Zuführleitung den Druck in der Kavität ermitteln und darauf eine Regelung basieren kann.

Nachteilig ist also, dass nur auf Basis eines mittelbar angekoppelten Drucksensors eine Nachlieferung des Fluid, welches durch die Absaugung an den medizintechnischen Instrumenten zur Verrringerung der Aufdehnung führt, erfolgen kann. Um dieses Problem zu lösen, gibt es im Stand der Technik den Ansatz, eine Aktivierung des mit der Absaugung verbundenen medizintechnischen Instrumentes zu erkennen und eine entsprechende Nachförderung bzw. Förderungserhöhung auszulösen. Die Erkennung erfolgt über den Strombedarf (z.B. EP 2 165 720) indem das medizintechnische Instrument direkt an eine Versorgungsbuchse der medizintechnische Geräte zur Bereitstellung eines Fluidmanagement angeschlossen ist. Andere Möglichkeiten den Betrieb des medizintechnischen Instrumentes zu erkennen, sind bekannt. Ein Nachteil dieser Lösung sind der hohe Aufwand, um den Betriebszustand zu erkennen, ohne jedoch Informationen über die Höhe der Leckage zu erhalten. Damit muss die Regelung der Absaugung, um ein Zusammenfallen der Kavität bei plötzlich einsetzendem höherem Abfluss (z.B. durch Shavereinsatz) zu verhindern, trotzdem auf weitere Sensoren oder Vorrichtungen für diese Kompensation zurückgreifen, da zwar der Zeitpunkt des Abflusses hierdurch bekannt ist, die Leckage-Menge jedoch vom Flusswiderstand der eingesetzten medizintechnischen Instrumente abhängt, die in der Regel ausgewechselt werden und nicht fest mit der Steuereinheit der medizintechnischen Instrumente verbunden sind.

Eine andere Schwierigkeit ist die Sicherstellung der Menge an Fluid im Durchfluss durch die Kavität bei gleichzeitig möglichst stabilen Druck. Die Durchflussmenge wird benötigt zur Entfernung von abgetragenem Gewebe und zur Gewährleistung der Sichtverhältnisse in der Kavität.

Die Durchflussmenge kann konstant gehalten werden und wird vom Operateur vorgewählt, oder soll einzeln oder zusätzlich auf Anforderung vom Operateur mit einer zeitweise oder dauerhaft erhöhten Rate (flush / wash) bereitgestellt werden. Sind Leckagen durch Öffnungen der aufgedehnten Kavität vorhanden, so entweicht Fluid und der Druck sinkt, so dass zwar der Durchfluss aufgrund der Menge des eingebrachten Fluides sichergestellt ist, aber ein Druck unter Umständen durch eine zu große Abflussmenge nicht aufrechterhalten werden kann.

Ein anderer Ansatz ist, den Druck konstant zu halten. Dadurch wird die Flussrate bei Leckagen sehr groß, da unter Umständen der Abflusswiderstand sehr niedrig bzw. die Querschnittsfläche für den Abfluss sehr groß wird, sobald Änderungen der Situation erfolgen, wie etwa zusätzliche Schnitte und Herausziehen von Instrumenten.

Eine andere Situation, wo eine solche Absaugung eingesetzt wird, erfordert die Bilanzierung der eingebrachten und abgesaugten Fluidmenge. Dies dient dazu, die Belastung des Patienten, bei dem die Kavität aufgedehnt ist, durch aufgenommenes Fluid abzuschätzen. Hierzu werden sowohl aus dem Patienten herauslaufende Anteile über Unterlagen mit Schlauchanschluss an ein Unterdruckgefäß und Trichter gesammelt, wie auch vom Boden unter der Behandlungsstelle die Fluidanteile über einen Bodensauger in einem Unterdruckgefäß gesammelt. Dieses Unterdruckgefäß ist an eine Absaugung angeschlossen, die den zur Beförderung zum Unterdruckgefäß notwendigen Unterdruck erzeugt. Ist eine der Zuführungen nicht mit Fluid gefüllt - wobei das Fluid in diesem speziellen Falle eine Flüssigkeit ist - sondern offen zur Atmosphäre, bricht der Unterdruck sehr schnell zusammen und andere ebenfalls an diese Unterdruckquelle angeschlossene Gefäße können das Fluid nicht befördern, da bevorzugt über den Weg des geringsten Widerstandes, der hier zwischen Unterdruckgefäß und Atmosphäre über die Beförderung von Luft gebildet wird, der Unterdruck ausgeglichen wird. Ein angeschlossenes medizintechnisches Instrument wird also in einer solchen Situation eines sich im Unterdruckzweig ausgleichenden Unterdruck nicht über die Absaugleitung zum Unterdruckgefäß kontrolliert und in erforderlichem Umfang durchströmt, sondern nur über das Fluid, welches durch den Druck der Aufdehnung hindurchgetrieben wird. Ist das medizintechnische Instrument ein mit bewegten Klingen arbeitendes Instrument, wo die Absaugung das abzutrennende Gewebe heranzieht (Shaver, Morcellator), so ist dessen Funktion beeinträchtigt. Eine Lösung ist, dann die Absauglinie mit der Verbindung zur Atmosphäre abzuklemmen bzw. ein darin eingebautes Ventil zu schließen und somit den Unterdurckausgleich zu unterbinden, wodurch aber die Bilanzierung durch die zwischenzeitliche Ansammlung der nicht abgesaugten Fluidmenge verfälscht wird, mindestens bis die Verbindung wieder hergestellt wird. Insgesamt ist das ein hoher Aufwand für den Operateur.

Weiterhin ist die zur Absaugung genutzte Fluidpumpe gemäß ihrer technischen Ausführung mit spezifischen Charakteristika versehen. Benutzt man eine Peristaltikpumpe, so sind die Flusscharakteristiken (Druck und Durchfluss) besser regelbar und eine Verstopfung der Absaugleitungen durch Gewebereste kann besser und schneller detektiert werden. Die Pumpvorrichtung an sich ist kleiner und erzeugt weniger Geräusche als eine Venturi- oder Diaphragma-Pumpe. Die Peristaltikpumpe kann als Flow-Senke betrachtet werden und ist vorteilhaft bei Bedarf an exaktem und sehr definierten Flow.

Vorteile der Venturi- oder Diaphragma-Pumpe sind ein Druckstoß-loser, gleichförmiger Fluss und eine sehr schnelle Anstiegszeit. Die Venturi- oder Diaphragma-Pumpe wird in dem Kontext der Erfindung in der minimal-invasiven Chirurgie typischerweise zur Erzeugung eines Unterdruckes mittels der Förderung von gasförmigem Fluid eingesetzt und benötigt für die Absaugung einen starren Unterdruckbehälter, worin das abgesaugte Fluid gesammelt wird. Andere Anwendungen sind denkbar, dann kommt die Pumpe aber mit dem abgesaugten Fluid in Kontakt und muss als Einwegprodukt ausgelegt werden oder gereinigt werden. Die Venturi- oder Diaphragma-Pumpe kann als Druck-Senke betrachtet werden und ist vorteilhaft bei hohem Flowwiderstand. Diese Art Pumpen wird als Unterdruck-erzeugende Pumpvorrichtung bezeichnet.

Die Peristaltikpumpe kann das abgesaugte Fluid direkt durch den eingelegten Schlauch in einen offenen Behälter pumpen, wirkt aber aufgrund ihres Funktionsprinzipes einen Schlauchabschnitt durch Rollen abzuquetschen auch als Sperre gegen einen angelegten Unterdruck. Somit kann auch die Persitaltikpumpe mit ihrer Förderseite an die Saugseite einer anderen Pumpe (z.B. den Unterdruckbehälter) angeschlossen werden.

Ein Konzept mit zwei verschiedenen Pumpvorrichtungen ist bekannt aus der WO93/17729. WO93/17729 offenbart eine Lösung von gekoppeltem großen und kleinen Unterdruckbehälter mit jeweils anderen daran angeschlossenen Pumpvorrichtungen zum Einsatz als Saug-Spül-Instrument in der intraokkularen Therapie. Diese Pumpvorrichtungen werden alternativ eingesetzt und sind über die Unterdruckbehälter an ein Saug-Spül-Instrument mit nur einer Absaugleitung angeschlossen. Das nur ein Instrument genutzt wird, wie in der WO93/17729 beschrieben mit verschiedenen Absaugpumpen-Charakteristika, ist der wesentliche Unterschied zu der dieser Erfindung zugrundeliegenden Situation. Die dieser Erfindung zugrundeliegenden Situation stellt verschiedene Anforderungen an die Absaugleitungen, dadurch dass diese jeweils an unterschiedlichen medizintechnischen Instrumenten angeschlossen sind. In der WO93/17729 beschrieben ist ein Saug-Spül-Instrument, welches in seinen Eigenschaften bekannt ist und fest mit der Schlauchkassette verbunden ist. In der dieser Erfindung zugrundeliegenden Situation sind erfindungsgemäß vielfache Konfigurationen der Nutzung einzelner Sauglinien vorteilhaft und die angeschlossenen medizintechnischen Instrumente sind erfindungsgemäß dem medizintechnischen Gerät zur Bereitstellung eines Fluidmanagement in ihren Eigenschaften nicht vorbekannt.

Ein Gerät aus dem Stand der Technik ist in WO2017/095679A1 offenbart.

### Erfindungsgemäße Lösung

Die erfindungsgemäße Lösung für ein medizintechnisches Gerät zur Bereitstellung eines Fluidmanagement zum Durchspülen einer Kavität besteht in einer Vorrichtung mit mindestens einer Pumpvorrichtung zur Flüssigkeitszufuhr und zwei Absaugvorrichtunen, die getrennt voneinander steuerbar sind, so wie in Anspruch 1 definiert.

Die Erfindung betrifft daher eine medizintechnische Vorrichtung zum Durchspülen von Kavitäten in der minimal-invasiven Chirurgie, enthaltend
(i) Vorratsbehälter (1) für die Spülflüssigkeit,
(ii) Zuführleitung (2) zur Zuführung von Spülflüssigkeit in die Körperhöhle (11),
(iii) gesteuerte Pumpe (3) zur Flüssigkeitszufuhr
(iv) gesteuerte erste Unterdruckpumpe (4)
(v) gesteuerte zweite Unterdruckpumpe (5)
(vi) erstes medizinisches Instrument (6) mit einer ersten Absaugleitung (7)
(vii) zweites medizinisches Instrument (8) mit einer zweiten Absaugleitung (9)
(viii) Abfallbehälter (10), verbunden mit erster Absaugleitung (7) und mit zweiter Absaugleitung (9).

In einer besonderen Ausführungsform der Erfindung ist der Abfallbehälter (10), verbunden mit der ersten Absaugleitung (7), mit der zweiten Absaugleitung (9) sowie mit der gesteuerten zweiten Unterdruckpumpe (5) durch die Leitung (12).

Weitere Ausführungsformen der Erfindung werden nachfolgend beschrieben.

Eine bevorzugte Pumpvorrichtung zur Flüssigkeitszufuhr besteht in einer Rollenradpumpe, wie sie im Stand der Technik in unzähligen Varianten beschrieben ist. Für die erfindungsgemäße Vorrichtung wird eine Pumpe verwendet, die einen Fluidfluß von 2.5 l/min fördern und einen Druck von 300 mmHg aufbauen kann. Alternativ kann für die Zwecke der Flüssigkeitszufuhr auch eine andere Verdrängerpumpe, wie beispielsweise eine Membranpumpe oder eine Impellerpumpe verwendet werden, sofern sie den gleichen Fluidfluß und Druck aufbauen können.

Für die Absaugvorrichtung können Rollenradpumpen, Venturipumpen oder Diaphragmapumpen verwendet werden. Ebenso sind alle Arten von Kolbenverdichter, Schraubenverdichter und Turboverdichter erfindungsgemäß.
- Rollenradpumpen wurden bereits oben bei der Darstellung des Standes der Technik beschrieben.
- In Diaphragmapumpen wird eine Trennmembran mechanisch bewegt um eine Kammer zyklisch zu vergrößern und zu verkleinern. Zwei im Ein- und Ausgang der Kammer angeordnete Ventile sorgen dafür, dass der Fluidstrom nur unidirektional stattfinden kann. Für den erfindungsgemäßen Zweck wird die Membran mittels eines bürstenlosen DC-Elektromotors angetrieben.
- In Venturipumpen (auch Strahlpumpen genannt) wird ein Treibstrahl verwendet, um die Pumpwirkung zu erzeugen. Der Treibstrahl kann aus Flüssigkeit (bevorzugt Wasser) oder Gas (z.B. Luft oder Stickstoff) bestehen. Für den erfindungsgemäßen Zweck wird bevorzugt Luft als Treibstrahl verwendet.

In jedem Fall muss die im Rahmen der Absaugvorrichtung verwendete Pumpe einen Unterdruck von 450 mmHg und einen Flüssigkeitsstrom von 1.5 l/min erzeugen können. Rollenrad- und Diaphragmapumpen haben den Vorteil sehr präzise steuerbar zu sein. Im Vergleich dazu sind Venturipumpen weniger präzise steuerbar.

Verbesserungen der Steuerbarkeit kann über eine Ventilsteuerung erzielt werden.

Die erfindungsgemäße Vorrichtung weist für die Absaugung zwei verschiedenartige Pumpen auf, z.B. eine Rollenradpumpe und eine Venturipumpe oder eine Rollenradpumpe und eine Diaphragmapumpe oder eine Venturipumpe und eine Diaphragmapumpe.

Besonders bevorzugt weist die erfindungsgemäße Vorrichtung für die Absaugung eine Rollenradpumpe und eine Venturipumpe oder eine Rollenradpumpe und eine Diaphragmapumpe auf.

Eine besondere Ausführungsform der Erfindung hat mindestens zwei Rollenräder, die im Sinne einer Persitaltikpumpe einen Schlauch abdrücken, und mindestens eine Pumpvorrichtung zur Erzeugung eines Unterdruck nach dem Venturi-Prinzip oder als Diaphragmapumpe. Ein Rollenrad (Inflow) wird genutzt, um die aufzudehnende Kavität mit einem Fluid zu beaufschlagen. Das Inflow-Rollenrad hat einen Sensor für die Umdrehungszahl. Ein Drucksensor ist an das Schlauchset über eine Membran oder eine ähnliche Lösung angekoppelt. Der Drucksensor erfasst mittelbar den Kavitätendruck. Die Umdrehung erfasst mittelbar die Fördermenge. Ein zweites Rollenrad (Outflow) kann ebenfalls einen Sensor für die Umdrehungszahl aufweisen und wird genutzt um mit mindestens einem Schlauch das Fluid aus der Kavität abzusaugen.

Eine Pumpvorrichtung zur Erzeugung eines Unterdruck nach dem Venturi-Prinzip oder als Diaphragmapumpe wird genutzt, um über einen Unterdruckbehälter Fluid aus dem Schlauch abzusaugen. Dies kann gleichzeitig oder unabhängig mit der Absaugung durch das Outflow-Rollenrad erfolgen.

Mit einer erfindungsgemäßen Vorrichtung kann im einfachsten Fall über eine Sauglinie der konstante Bedarf an Fluidfluß für die Spülung (Durchflussmenge) und über mindestens eine weitere Sauglinie ein erhöhter Bedarf an Fluidfluß für die zusätzliche Spülung realisiert werden. Hierzu sind im einfachsten Fall keine Ventile notwendig, da die Peristaltikpumpe dicht ist und die Unterdruck-erzeugende Pumpvorrichtung soweit reduziert werden kann, dass nichts abgesaugt wird. Der Vorteil gegenüber einer reinen Doppelrollenpumpe ergibt sich aus der einfachen Erzeugung eines hohen Vordruckes zum Saugen durch einen hohen Flowwiderstand und der Vermeidung von Schlupf am Rollenrad bei hohen Geschwindigkeiten, wodurch die Fördermengen unkontrolliert schwanken. Die hohen Geschwindigkeiten werden notwendig, wenn ein hoher Vordruck für die Absaugung erzeugt werden muss. Mit Vordruck ist in diesem Fall der Unterdruck an der Pumpvorrichtung gemeint, der durch das Absaugen mit der Vakuumpumpe erreicht wird. Aufgrund des Druckgefälles zwischen Gelenkshöhle und Unterdruckbehälter ergibt sich ein Durchfluss, der vom Flowwiderstand des verwendeten Instrumentes abhängt.

Die grundlegende Funktion eines Durchflusses bei stabilem Druck in der Kavität kann in einem Ausführungsbeispiel durch die Kombination von Inflow-Rollenrad mit der Absaugung über die Vakuumpumpe realisiert werden. Hier wird als Input für die Regelung der vorgegebene Druck in der Kavität nach dem Stand der Technik geregelt. Als Input für die Regelung erfasst ein Sensor über eine in Fluidkontakt mit der Kavität stehenden Membran den Fluiddruck. Dieser Sensor ist im Fluidpfad hinter dem Inflow-Rollenrad positioniert. Ein konstanter Durchfluss wird erzeugt durch Kopplung dieses Inflow-Rollenrades mit dem durch den erzeugten Unterdruck abgeführten Volumen. Sind Zufluss und Abfluss gleich, ergibt sich ein Durchfluss bei einem stabilen Kavitätendruck. Kommen weitere Aktionen vom Operatuer, wie z.B. einen höheren Durchfluss anzufordern bzw. werden zusätzliche Sauglinien geöffnet. So kann beispielsweise das Outflow-Rollenrad benutzt werden, um einen entsprechenden (zusätzlichen) Unterdruck in einer weiteren Sauglinie, die ebenfalls am Unterdruckbehälter angeschlossen ist, zu erzeugen. Dieser vom Outflow-Rollenrad erzeugte Unterdruck wird direkt vom Inflow-Rollenrad kompensiert, beispielsweise indem die Drehzahl des Inflow-Rollenrades um die Drehzahl des Outflow-Rollenrades erhöht wird. Andere Lösungen, die den geförderten Volumenstrom besser approximieren, sind ebenfalls denkbar. Das je Zeiteinheit geförderte Volumen (Volumenstrom) kann sich durch Schlauchdurchmesser und Dicke der Schlauchwandung sowie das benutzt Material ändern. Durch die direkte Volumenerhöhung der Inflow-Seite in Antwort der Aktivierung des Outflow-Rollenrades wird ein Druckeinbruch durch eine größere Entnahme von Fluid aus der Kavität wirkungsvoll verhindert.

Andere Ausgestaltungen mit mindestens 2 Sauglinien als einzeln ansteuerbare Peristaltikpumpen, oder mit mindestens 2 Sauglinien mit getrennt regelbaren Diaphragma- oder Venturi-Pumpen oder andere Kombinationen sind ebenfalls erfindungsgemäß.

### Ausführungsbeispiele

Die vorliegende Erfindung wird durch die nachfolgenden Beispiele weiter erläutert, ohne dass durch die Beispiele eine Beschränkung vorgenommen werden soll. Der Fachmann auf dem Gebiet kann anhand der vorliegenden Beschreibungen und seiner Erfahrungen auf dem Gebiet weitere Ausführungsformen der Erfindung entwickeln, ohne erfinderisch werden zu müssen.

Eine mögliche Konfiguration der Pumpvorrichtungen und der Kavität kann dergestalt sein, dass die Inflow-Pumpe über eine Zuführleitung an das Endoskop zur Betrachtung der Kavität angeschlossen ist und an das Outflow-Rollenrad ist auf der Unterdruckseite sowohl ein HF-Instrument, wie auch ein medizintechnisches Instrument mit bewegten Klingen (z.B. Shaver) angeschlossen ist, wobei letztere über eine Abklemmvorrichtung oder ein Ventil für den einzelnen Schlauch angesteuert werden können. Die Unterdruck-erzeugende Pumpvorrichtung ist über den Unterdruckbehälter direkt an die Kavität angeschlossen (dritte Saugleitung, siehe Absatzende). Das Outflow-Rollenrad ist auf der Überdruckseite ebenfalls an den Unterdruckbehälter angeschlossen, welcher als Sammelbehälter dient und entsprechend so groß ausgelegt sein kann, dass er während einer typischen Prozedur nicht gewechselt oder geleert werden muss. Dere Saugzweig am Rollenrad ist in Betrieb und Stillstand geschlossen, so dass der Unterdruck durch den Betriebszustand des Rollenrades nicht beeinflusst wird. Die zwei Saugleitungen am Outflow-Rollenrad können über Abklemmvorrichtungen einzeln okkludiert werden, so dass die Zustände
1-0 (Unterdruck-Pumpe offen - Outflow Rollenrad gestoppt),
0-1 (Unterdruck-Pumpe geschlossen - Outflow Rollenrad in Bewegung),
1-1 (Unterdruck-Pumpe offen - Outflow Rollenrad in Bewegung) per Abklemmvorrichtung und der Zustand
0-0 (Unterdruck-Pumpe geschlossen - Outflow Rollenrad gestoppt),
über Stillstand des Rollenrades erzeugt werden kann.

Der Zustand der dritten Saugleitung kann über eine Ansteuerung der Vakuumpumpe geregelt werden.

Vorteilhaft ist es die Abklemmvorrichtung zwischen Outflow-Rollenrad und medizintechnischem Instrument anzuordnen und über eine dem Operateur zugängliche Auslösevorrichtung anzusteuern. Diese Lösung ermöglicht dem Gerät zur Bereitstellung eines Fluidmanagement den Zustand der Abklemmvorrichtung zu erfassen und in die Druck- und Durchfluss-Regelung einzubeziehen.

**Beispiel 1:** In der oben skizzierten Ausführungsform kann bei Einsatz der medizintechnischen Instrumente (beispielhaft Shaver oder HF) ein In-Outflow-Management erfolgen, ohne von der Erfassung der Betriebszustände der medizintechnischen Instrumente abhängig zu sein. Die im Gerätekonzept des Gerätes zur Bereitstellung eines Fluidmanagement vorgesehene Outflow-Rollenradpumpe und die Unterdruck-erzeugenden Pumpvorrichtung können getrennt ansteuerbar für die Sauglinien, also die Fluidverbindungen von der Kavität zum Gerät, die jeweils erforderlichen Unterdrücke und Flussraten bereitstellen. Beide Pumpvorrichtungen sind regelbar, wobei das Outflow-Rollenrad beispielsweise eine höhere Absaugung von medizintechnischem Instrument (z.B. Shaver/HF) übernehmen kann und die Unterdruck-erzeugende Pumpvorrichtung einen kontinuierlichen Durchfluss sicherstellt. Als großer Vorteil für den Operateur entfällt das Umstöpseln bzw. Schließen des Hahns am Trokar, der typisch genutzten Hülse für das Einführen des Endoskops.

Im Szenario einer Arthroskopie werden in diesem Beispiel schnelle Druckänderungen vermieden, da bei Lösungen gemäß dem Stand der Technik bei der Öfnung der Saugleitungen zu einem medizintechnischen Instrument (z.B. Shaver/HF) durch den auftretenden Druckabfall am Sensor am Inflow-Rollenrad ein hoher Bedarf an Nachförderung entsteht und erst relativ spät gegengesteuert werden kann, wodurch sich durch die Druckspitzen eine größere Extravasation des Fluides ergibt, als mit der erfindungsgemäßen Lösung. Die Extravasation führt zu nachteiligen Schwellungen im Umfeld der aufgedehnte Kavität. Diese Ausführungsform der erfindungsgemäßen Lösung ermöglicht erstmals eine "Low pressure Arthroscopy" mit etwas über 30mmHg Kavitätendruck im Gelenk, da mit den Vorrichtungen gemäß Stand der Technik die Druckschwankungen zu groß sind und die Kavität kollabiert.

**Beispiel 2:** In der oben skizzierten Ausführungsform kann ein medizintechnisches Instrument mit hohem Bedarf an Durchflussmenge (z.B. ein Shaver, Morzellator oder andere Geräte, die Gewebestücke abtrennen und/oder absaugen) direkt an das Outlow-Rollenrad angeschlossen werden, welches in den Unterdruckbehälter fördert, und ein medizintechnisches Instrument mit hohem Flowwiderstand (z.B. ein HF-Instrument) an die Unterdruck-erzeugende Pumpvorrichtung sowie über die gleiche Sauglinie an die Kavität. Das medizintechnische Instrument mit hohem Flowwiderstand wird über ein Ventil abgeklemmt und nur bei Aktivierung wird dort die Sauglinie geöffnet.

Der Vorteil ist eine schnelle Absaugungswirkung am medizintechnischen Instrument mit hohem Flowwiderstand bei einem sehr stabilen Kavitätendruck und einem unabhängig vom Kavitätendruck einstellbaren Durchfluss. Zusätzlich hat dieses Beispiel die Vorteile für den Operateur, das ein Umstöpseln bzw. Schließen des Hahns am Trokar entfällt.

**Beispiel 3:** In der oben skizzierten Ausführungsform kann das Outflow-Rollenrad direkt an die Kavität angeschlossen sein und die Unterdruck-erzeugende Pumpvorrichtung an ein medizintechnisches Instrument mit bewegten Klingen (z.B. Shaver, Morzellator oder andere Geräte, die Gewebestücke abtrennen und/oder absaugen). Somit kann der Kavitätendruck gut kontrolliert und der Zu- und Abfluss gut geregelt werden. Wenn das medizintechnische Instrument mit bewegten Klingen aktiviert wird, kommt es aufgrund des Pumpprinzipes zu keinen Druckschwankungen und eine stabile Kavitätenwandung ist dadurch gegeben. Dies ist besonders hilfreich in Kavitäten mit sehr flexiblen Wandungen,wie eine Gebärmutter.

**Beispiel 4:** In der oben skizzierten Ausführungsform kann das Outflow-Rollenrad und das Inflow-Rollenrad direkt an ein Endoskop mit separierten Kanälen für In- und Outflow angeschlossen sein, welches sich in der Kavität befindet zur Erzeugung eines kontinuierlichen Durchflusses. Die Unterdruck-erzeugende Pumpeinheit kann angeschlossen sein an ein Sauginstrument oder über ein Ventil abtrennbar zusätzlich an das Endoskop. In der Urologie bei der Steinentfernung im Bereich der Harnleiter oder dem Nierenkelch wird der Stein mit einem Instrument fixiert, welches den Stein umgreift (Steinfängerkorb). Der Vorgang der Umgreifung kann durch per Aktivierung der Sauglinie mit der Unterdruck-erzeugende Pumpeinheit aufgegebenem zusätzlichen Saugimpuls befördert werden, indem durch die zusätzliche Absaugung der Stein in dem Instrument positioniert wird. Diese impulsartige Saugsteigerung ist sehr vorteilhaft, da die Situation in der Kavität nicht geändert wird und nur ein zusätzlicher Sog in Richtung der Absaugungsöffnung erzeugt wird, dessen Wirkung durch vermehrten Zufluss ausgeglichen werden kann. Aufgrund der Schlauchlänge und Durchmesser (also Flowwiderstand) wird ein enormer Vordruck benötigt, der mit dem Pumpenprinzip der unterdruckerzeugenden Pumpeinheit am besten erzeugt werden kann. Im Normalbetrieb wird eine exakte Druckregelung über kleinste Mengen benötigt (Gefahr der Schädigung der Nierenkelch-Region), was mit Peristaltikpumpen am besten erfolgen kann, diese können aber für eine Saugimpuls nicht den nötigen Vordruck schnell genug erzeugen, bzw. wären weit überdimensioniert wenn sie das täten.

### Bezugszeichenliste

(1) Vorratsbehälter für die Spülflüssigkeit,
(2) Zuführleitung zur Zuführung von Spülflüssigkeit in die Körperhöhle (11)
(3) gesteuerte Pumpe zur Flüssigkeitszufuhr
(4) gesteuerte erste Unterdruckpumpe
(5) gesteuerte zweite Unterdruckpumpe
(6) erstes medizinisches Instrument
(7) erste Absaugleitung am ersten medizinischen Instrument
(8) zweites medizinisches Instrument
(9) zweite Absaugleitung am zweites medizinisches Instrument
(10) Abfallbehälter
(11) Körperhöhle
(12) Leitung von Abfallbehälter zur gesteuerten zweiten Unterdruckpumpe.

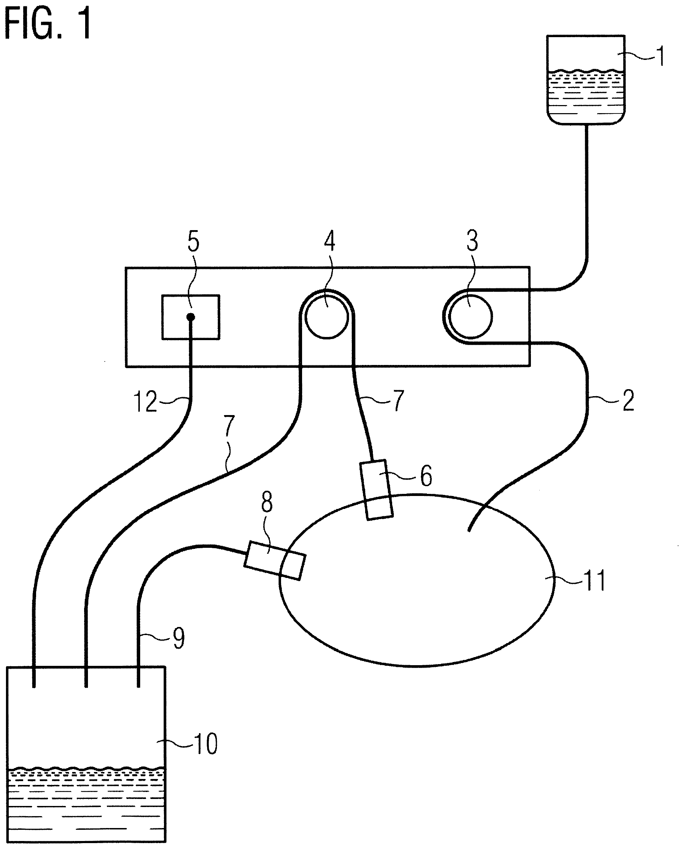

## Patentansprüche

1. Medizintechnische Vorrichtung zum Durchspülen von Kavitäten in der minimal-invasiven Chirurgie, enthaltend
(i) Vorratsbehälter (1) für die Spülflüssigkeit,
(ii) Zuführleitung (2) zur Zuführung von Spülflüssigkeit in die Körperhöhle (11),
(iii) gesteuerte Pumpe (3) zur Flüssigkeitszufuhr
(iv) gesteuerte erste Unterdruckpumpe (4)
(v) gesteuerte zweite Unterdruckpumpe (5)
(vi) erstes medizinisches Instrument (6) mit einer ersten Absaugleitung (7)
(vii) zweites medizinisches Instrument (8) mit einer zweiten Absaugleitung (9)
(viii) Abfallbehälter (10), verbunden mit erster Absaugleitung (7) und mit zweiter Absaugleitung (9),
**dadurch gekennzeichnet, dass** die gesteuerte erste Unterdruckpumpe (4) und die gesteuerte zweite Unterdruckpumpe (5) verschiedenartig sind.

2. Medizintechnische Vorrichtung gemäß Anspruch 1, **dadurch gekennzeichnet, dass** der Abfallbehälter (10), verbunden ist mit der ersten Absaugleitung (7), mit der zweiten Absaugleitung (9) sowie mit der gesteuerten zweiten Unterdruckpumpe (5) durch eine Leitung (12).

3. Medizintechnische Vorrichtung gemäß Anspruch 1 oder 2, **dadurch gekennzeichnet, dass** die gesteuerte Pumpe (3) zur Flüssigkeitszufuhr eine Rollenradpumpe ist.

4. Medizintechnische Vorrichtung gemäß Anspruch 1, 2 oder 3, **dadurch gekennzeichnet, dass** die gesteuerte erste Unterdruckpumpe (4) und/oder die gesteuerte zweite Unterdruckpumpe (5) eine Rollenradpumpe ist.

5. Medizintechnische Vorrichtung gemäß mindestens einem der Ansprüche 1 bis 4, **dadurch gekennzeichnet**, gesteuerte erste Unterdruckpumpe (4) eine Rollenradpumpe und die gesteuerte zweite Unterdruckpumpe (5) eine Venturipumpe ist.

6. Medizintechnische Vorrichtung gemäß mindestens einem der Ansprüche 1 bis 4, **dadurch gekennzeichnet**, gesteuerte erste Unterdruckpumpe (4) eine Rollenradpumpe und die gesteuerte zweite Unterdruckpumpe (5) eine Diaphragmapumpe ist.

## Claims

1. A medical device for rinsing cavities during minimally invasive surgery, comprising:
(i) a reservoir (1) for the rinsing liquid,
(ii) a feed line (2) for feeding rinsing liquid into the body cavity (11),
(iii) a controlled pump (3) for feeding liquid,
(iv) a controlled first vacuum pump (4),
(v) a controlled second vacuum pump (5),
(vi) a first medical instrument (6) with a first suction line (7),
(vii) a second medical instrument (8) with a second suction line (9),
(viii) a waste container (10) connected to the first suction line (7) and the second suction line (9),
**characterized in that** the controlled first vacuum pump (4) and the controlled second vacuum pump (5) are different.

2. The medical device according to Claim 1, **characterized in that** the waste container (10) is connected to the first suction line (7), the second suction line (9), and the controlled second vacuum pump (5) through a line (12).

3. The medical device according to Claim 1 or 2, **characterized in that** the controlled pump (3) for feeding liquid is a roller wheel pump.

4. The medical device according to Claim 1, 2, or 3, **characterized in that** the controlled first vacuum pump (4) and/or the controlled second vacuum pump (5) is a roller wheel pump.

5. The medical device according to at least one of the Claims 1 to 4, **characterized in that** the controlled first vacuum pump (4) is a roller wheel pump and the controlled second vacuum pump (5) is a Venturi pump.

6. The medical device according to at least one of the Claims 1 to 4, **characterized in that** the controlled first vacuum pump (4) is a roller wheel pump and the controlled second vacuum pump (5) is a diaphragm pump.

## Revendications

1. Dispositif médical destiné à rincer des cavités dans la chirurgie minimalement invasive, comprenant
(i) un réservoir (1) pour le liquide de rinçage,
(ii) une conduite d'alimentation (2) pour alimenter le liquide de rinçage dans la cavité corporelle (11),
(iii) une pompe commandée (3) pour l'alimentation en liquide
(iv) une première pompe à vide commandée (4)
(v) une seconde pompe à vide commandée (5)
(vi) un premier instrument médical (6) avec une première conduite d'aspiration (7)
(vii) un second instrument médical (8) avec une seconde conduite d'aspiration (9)
(viii) un contenant à déchets (10), relié à la première conduite d'aspiration (7) et à la seconde conduite d'aspiration (9),
**caractérisé en ce que** la première pompe à vide commandée (4) et la seconde pompe à vide commandée (5) sont différentes.

2. Dispositif médical selon la revendication 1, **caractérisé en ce que** le contenant à déchets (10) est relié à la première conduite d'aspiration (7), à la seconde conduite d'aspiration (9) ainsi qu'à la seconde pompe à vide commandée (5) par une conduite (12).

3. Dispositif médical selon la revendication 1 ou 2, **caractérisé en ce que** la pompe commandée (3) pour l'alimentation en liquide est une pompe à rouleaux.

4. Dispositif médical selon la revendication 1, 2 ou 3, **caractérisé en ce que** la première pompe à vide commandée (4) et/ou la seconde pompe à vide commandée (5) est une pompe à rouleaux.

5. Dispositif médical selon au moins une des revendications 1 à 4, **caractérisé en ce que** la première pompe à vide commandée (4) est une pompe à rouleaux et la seconde pompe à vide commandée (5) est une pompe Venturi.

6. Dispositif médical selon au moins une des revendications 1 à 4, **caractérisé en ce que** la première pompe à vide commandée (4) est une pompe à rouleaux et la seconde pompe à vide commandée (5) est une pompe à diaphragme.
